# EUROPÄISCHE PATENTSCHRIFT

(11) **EP 1 850 818 B1**
(45) Veröffentlichungstag und Bekanntmachung des Hinweises auf die Patenterteilung: **31.12.2014**
(21) Anmeldenummer: 05824388.2
(22) Anmeldetag: 25.11.2005
(51) Int. Cl.: A61M 1/00, D04B 21/16, A61F 13/00

(54) **VORRICHTUNG ZUR BEHANDLUNG VON WUNDEN**
WOUND TREATMENT DEVICE
DISPOSITIF DE TRAITEMENT DE PLAIES

(30) Priorität: 15.02.2005 DE 102005007016
(43) Veröffentlichungstag der Anmeldung: 07.11.2007
(73) Patentinhaber: Biowim Products GmbH, 79199 Kirchzarten (DE)
(72) Erfinder: Wilhelm Fleischmann, 79104 Freiburg (DE)
(74) Vertreter: Patentanwälte Westphal, Mussgnug & Partner
(86) Internationale Anmeldenummer: PCT/EP2005/012621
(87) Internationale Veröffentlichungsnummer: WO 2006/087021

(56) Entgegenhaltungen:
- EP-A- 0 880 953
- DE-A1- 19 832 634
- GB-A- 2 292 526
- GB-A- 2 299 349
- US-A- 5 589 245
- US-B1- 6 755 052

## Beschreibung

Die Erfindung betrifft eine Vorrichtung zur Behandlung von Wunden gemäß dem Oberbegriff des Patentanspruchs 1.

Aus der EP 0 620 720 B1 ist es bekannt, auf die Wundoberfläche einer Wunde eine Wundeinlage aufzubringen, die aus einem offenzelligen Polymer-Schaumstoff besteht. Die Wundfläche und die Wundeinlage werden durch eine Abdeckung abgedichtet, die auf der die Wundfläche umgebenden Haut abdichtend befestigbar ist. Über eine Absaugleitung kann unter der Abdeckung ein Unterdruck erzeugt werden, um Gewebeflüssigkeit aus der Wunde abzusaugen. Der Polymer-Schaumstoff weist eine relative geringe Porengröße auf, um ein Einwachsen des Wundgewebes in die Wundeinlage zu verhindern. Der kleinporige Schaumstoff kann sich allerdings z.B. durch Gerinsel zusetzen, so dass die Absaugwirkung entsprechend nachläßt und die Wundeinlage gewechselt werden muss.

Aus der US 4 382 441 ist es bekannt, eine Wundeinlage aus einem offenporigen Schaumstoff oder einem textilen Gewebe auf die Wundoberfläche aufzubringen und durch eine Abdeckung abzuschließen, die rings um die Wunde abdichtend auf der Hautoberfläche befestigt wird. In die Wundeinlage führen Schlauchleitungen, die als Zuführleitung und als Absaugleitung ausgebildet sind. Über diese Schlauchleitungen kann eine Behandlungsflüssigkeit durch die Wundeinlage geleitet werden, um mit der Wundoberfläche in Kontakt zu kommen. Ein Unterdruck wird dabei in der Wundeinlage nicht erzeugt. Das Problem des Zusetzens der Wundeinlage kann auch hier auftreten.

Gemäß der EP 0 880 953 B1 wird diese Vorrichtung dahingehend verbessert, dass die Zuführleitung und die Absaugleitung unabhängig voneinander steuerbar sind, so dass die zugeführte Behandlungsflüssigkeit über eine einstellbare Zeitdauer auf die Wunde einwirken kann, während Zeitintervalle dazwischengeschaltet werden können, in denen keine Behandlungsflüssigkeit auf die Wunde einwirkt und gegebenenfalls auch ein Unterdruck erzeugt werden kann. Ein solcher Unterdruck übt eine Zugwirkung auf die Gewebezellen der Wunde aus, wodurch das Zellwachstum und die Gewebeproliferation begünstigt werden.

Der Erfindung liegt die Aufgabe zugrunde, eine Vorrichtung zur Behandlung von Wunden zur Verfügung zu stellen, die den Heilungsprozess begünstigt.

Diese Aufgabe wird erfindungsgemäß gelöst durch eine Vorrichtung mit den Merkmalen des Patentanspruchs 1.

Vorteilhafte Ausführungen und Weiterbildungen der Erfindung sind in den Unteransprüchen angegeben.

Der wesentliche Gedanke der Erfindung besteht darin, eine Wundeinlage aus einem flächigen textilen Abstandsmaterial zu verwenden. Ein solches Abstandsmaterial weist wenigstens zwei Oberflächenschichten auf, zwischen denen sich Abstandsfäden befinden, die diese Oberflächenschichten elastisch federnd beabstandet halten.

Solche flächigen textile Abstandsmaterialien (spacer fabrics) werden insbesondere als Abstandsgewirke hergestellt. Sie zeichnen sich durch Flexibilität, Komprimierbarkeit und ein hohes Raumvolumen bei geringem Gewicht aus (z. B. "Kettenwirk-Praxis 2/2001, Seiten 47/48").

Abstandsgewirke werden als Oberflächenschicht bei Sitz- und Liegemöbeln verwendet und als Auspolsterung in der Bekleidungsindustrie.

In diesem Zusammenhang wurde bereits vorgeschlagen (Exogenous Dermatology 2002; 1, Seiten 276 bis 278), Patienten auf einen textilen Abstandsgewirke zu lagern, um die Entstehung von Druckgeschwüren (Dekubitus) zu verringern.

Weiter ist es aus der DE 198 32 634 Al bekannt, zur Behandlung von Leistenbrüchen Implantate aus einem flächigen textilen Abstandsmaterial zwischen Bauchwand und Peritoneum einzusetzen.

Bei der erfindungsgemäßen Vorrichtung wird das flächige textile Abstandsmaterial als Wundeinlage verwendet, wobei wenigstens eine Oberflächenschicht des Abstandsmaterials mit der Wundoberfläche in Berührung kommt. Die mit der Wundoberfläche in Berührung kommende Oberflächenschicht weist eine biokompatible Oberfläche auf, um eine Verträglichkeit mit dem Wundgewebe zu gewährleisten. Weiter weist diese Oberflächenschicht eine Struktur auf, die zum einen das Durchtreten von Flüssigkeit ermöglicht und zum anderen das Einwachsen des Wundgewebes verhindert.

Die Wundeinlage ermöglicht eine erhebliche Verbesserung der Wundbehandlung und der Wundheilung. Zwischen den Oberflächenschichten wird ein großvolumiger Zwischenraum gebildet, in welchem sich nur die Abstandsfäden befinden. Gewebeflüssigkeit kann durch die poröse Oberflächenschicht in den Zwischenraum eindringen und dort aufgenommen und gespeichert werden. Wird die Wundeinlage durch eine Überdeckung z.B. ein Verbandsmaterial oder eine Abdichtfolie in der Wunde komprimiert, so übt die Wundeinlage einen Druck auf das Wundgewebe aus, der die Gewebeflüssigkeit aus dem Wundgewebe presst, was den Heilungsprozess zusätzlich begünstigt.

Vorzugsweise kommuniziert mit dem großvolumigen Zwischenraum wenigstens eine Schlauchleitung, die insbesondere als Absaugleitung ausgebildet ist. Gewebeflüssigkeit, die aus der Wunde über die poröse Oberflächenschicht in den Zwischenraum eindringt, kann dort abgesaugt werden, wobei aufgrund des offenen Volumens des Zwischenraums ein Zusetzen praktische ausgeschlossen ist. Für das Absaugen ist es besonders vorteilhaft, wenn die Überdeckung durch eine Abdichtfolie gebildet ist, welche die Wunde mit der Wundeinlage luftdicht überdeckt und ringsum die Wunde auf der Haut abgedichtet befestigt wird, wie dies von der sogenannten Vakuumversiegelung bekannt ist. Selbstverständlich ist es in gleicher Weise möglich, über den Zwischenraum Behandlungsflüssigkeit über eine Zuführleitung zuzuführen, die dann durch die Oberflächenschicht an das Wundgewebe gelangt. Durch gesteuertes Zuführen von Behandlungsflüssigkeit und Absaugen der Behandlungsflüssigkeit und des Wundsekrets ist eine optimale Wundbehandlung möglich.

Die hohe Kompressibilität des textilen Abstandsmaterials ermöglicht in Verbindung mit einer Abdichtfolie weiter eine vorteilhafte Einwirkung auf die Wundheilung und die Gewebsproliferation. Die Wundeinlage wird in die Wunde eingebracht, worauf mittels einer Pumpe über die Absaugleitung ein Unterdruck unter der Abdichtfolie erzeugt wird. Durch diese Saugwirkung wird zum einen das Abstandsmaterial gegen die Elastizität der Abstandsfäden komprimiert. Zum zweiten wird auf das Gewebe der Wunde eine Saugwirkung ausgeübt, wodurch Zugkräfte auf die Zellen des Gewebes wirken. Wird der Saugvorgang beendet, z. B. indem ein in die Absaugleitung eingeschaltetes Ventil geschlossen wird, so bleibt zunächst der Unterdruck unter der Abdichtfolie erhalten. Durch die elastische Rückstellkraft des Abstandsmaterials übt dieses einen Flächendruck auf das Wundgewebe aus. Dieser Druck bewirkt zum einen einen Druck auf die Zellen des Wundgewebes. Zum anderen wird Gewebsflüssigkeit aus den Zwischenzellräumen ausgepreßt und gelangt durch die poröse Oberflächenschicht des Abstandsmaterials in den offenen Zwischenraum dieses Abstandsmaterials. Das auf die Zellen abwechselnde Einwirken von Zugkräften während der Absaugphase und von Druckkräften während der Expansionsphase des Abstandsmaterials stimuliert die Gewebeneubildung besonders intensiv. Dies beruht zum einen darauf, dass die Zellen zyklisch in zueinander senkrechten Richtungen deformiert werden. Zum zweiten wird ein Zellwachstum dadurch gefördert, dass während der Druckphase die Zelldurchblutung reduziert wird bis zur Ischämie, während bei nachlassendem Druck eine reaktive Hyperämie und verstärkte Blutreperfusion erfolgt. Zum dritten wird die sich zwischen den Zellen befindende Gewebeflüssigkeit durch die Zug- und Druckwirkung verstärkt in Bewegung gesetzt und quasi aus dem Gewebe "gemolken".

Bei tiefen Wunden kann die Wundeinlage so in den Wundspalt eingelegt werden, dass sie mit beiden Oberflächen an der Wundoberfläche anliegt. Die elastische Rückstellkraft des Abstandsmaterials drückt dabei die beiden Oberflächen dieses Abstandsmaterials auseinander und somit gegen die einander gegenüberliegenden Wundoberflächen. Die Abdichtfolie hat hierbei nur die Aufgabe, die Wunde zu verschließen, um einen Unterdruck in der Wunde erzeugen zu können.

Bei oberflächlichen Wunden wird die Wundeinlage auf die Wundoberfläche aufgelegt, so dass nur eine Oberflächenschicht mit der Wundoberfläche in Berührung kommt, während die entgegengesetzte Oberflächenschicht an der Abdichtfolie anliegt. Die elastische Rückstellkraft des Abstandsmaterials stützt sich in diesem Falle an der Abdichtfolie ab. Die Abdichtfolie muss hierzu ausreichend straff gespannt sein oder eine ausreichende Flächensteifigkeit aufweisen.

Das flächige Textilabstandsmaterial kann in unterschiedlicher Weise hergestellt werden. Wichtig ist dabei insbesondere, dass durch elastisch federnde Abstandfäden ein ausreichend großvolumiger Zwischenraum gebildet wird und das Abstandsmaterial die gewünschten elastischen Eigenschaften aufweist. Weiter ist wichtig, dass die mit der Wunde in Berührung kommende Oberflächenschicht gewebeverträglich ist, das Durchtreten von Flüssigkeit ermöglicht und das Einwachsen des Wundgewebes verhindert. Diese Kriterien können in verschiedener Weise erfüllt werden.

Der Werkstoff und die Dimensionierung der Abstandsfäden wird gemäß den gewünschten mechanischen Eigenschaften gewählt, d.h. im Hinblick auf die Dicke des Abstandsmaterials bzw. des Abstandes der Oberflächenschichten und im Hinblick auf die gewünschte Federkraft. Die mit der Wunde in Berührung kommende Oberflächenschicht kann aus einem anderen Werkstoff bestehen. Dabei kann das Abstandsmaterial als Abstandsgewirke ausgebildet sein, bei welchem die Abstandsfäden in die die Oberflächenschicht bildende textile Lage eingewirkt sind.

Die Oberflächenschicht kann dabei eine textile Stofflage aus einem biokompatiblen Werkstoff sein, wobei die geringe Dicke und die kleine Maschenweite dieser Stofflage die gewünschte Struktur gewährleisten.

In einer anderen Ausführung kann die Oberflächenschicht, die mit den Abstandsfäden zusammengewirkt ist, aus einem Werkstoff bestehen, der nach mechanischen und textiltechnischen Eigenschaften gewählt ist. Diese Oberflächenschicht weist dabei die Struktur auf, die das Durchtreten von Flüssigkeit ermöglicht und das Einwachsen des Wundgewebes verhindert. Um die Biokompatibilität und Gewebeverträglichkeit der Oberflächenschicht zu gewährleisten, wird diese mit einem biokompatiblen Werkstoff beschichtet. Die Beschichtung darf dabei die Struktur der Oberflächenschicht nicht wesentlich beeinträchtigen.

Weiter ist eine Ausführung möglich, bei welcher die Oberflächenschicht eine großmaschige Stützlage aufweist, in welche die Abstandsfäden eingewirkt sind. Der Werkstoff der Stützlage kann dabei nach seinen mechanischen und textiltechnischen Eigenschaften ausgewählt werden. Diese Stützlage wird mit einer Decklage aus dem biokompatiblen Werkstoff kaschiert. Diese Decklage kann beispielsweise ein entsprechend kleinporiger Schaumstoff sein, wobei die geringe Schichtdicke des Schaumstoffes ein Zusetzen vermeidet. Die Decklage kann gegebenenfalls auch einen Wirkstoff enthalten und z.B. als Medikamententräger dienen.

Als biokompatibler Werkstoff wird vorzugsweise ein polymerer Kunststoff, insbesondere ein Polyvinylalkohol verwendet.

Im Folgenden wird die Erfindung anhand von in der Zeichnung dargestellten Ausführungsbeispielen näher erläutert. Es zeigen:
Figur 1
   schematisch den Aufbau der Wundeinlage,
Figur 2
   ein erstes Anwendungsbeispiel mit komprimierter Wundeinlage,
Figur 3
   dieses Anwendungsbeispiel mit expandierender Wundeinlage,
Figur 4
   ein zweites Anwendungsbeispiel zur Behandlung einer Flächenwunde unter Vakuumversiegelung mit komprimierter Wundeinlage,
Figur 5
   das zweite Anwendungsbeispiel mit expandierender Wundeinlage,
Figur 6
   ein drittes Anwendungsbeispiel zur Behandlung einer tiefen Wunde mit komprimierter Wundeinlage und
Figur 7
   das dritte Anwendungsbeispiel mit expandierender Wundeinlage.

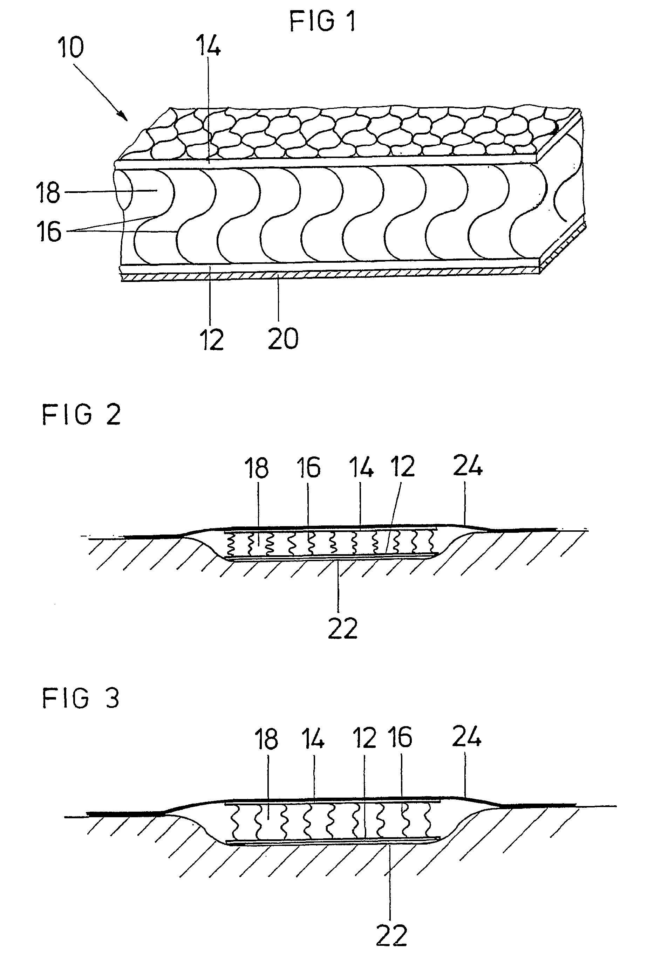

Erfindungsgemäß wird eine Wundeinlage 10 verwendet, deren Aufbau in einem Ausführungsbeispiel schematisch in Figur 1 dargestellt ist. Die Wundeinlage 10 besteht aus einem flächigen textilen Abstandsmaterial und wird entsprechend den jeweiligen Erfordernissen der zu behandelnden Wunde auf eine geeignete Größe zugeschnitten. Gegebenenfalls kann die Wundeinlage auch in vorgegebenen Standardabmessungen vorrätig gehalten werden. Das textile Abstandsmaterial weist eine erste Oberflächenschicht 12 und eine zweite Oberflächenschicht 14 auf. Die Oberflächenschichten 12 und 14 werden durch elastisch federnde Abstandsfäden 16 voneinander beabstandet gehalten. Dadurch wird zwischen den Oberflächen 12 und 14 ein Zwischenraum 18 gebildet, der relativ großvolumig ist. In diesem Zwischenraum 18 befinden sich nur die Abstandsfäden 16, die einen relativ großen gegenseitigen Abstand aufweisen, so dass der Zwischenraum 18 in der Flächenebene der Wundeinlage 10 weitgehend offen ist und zwischen den Abstandsfäden 16 einen großen freien Durchtrittsquerschnitt aufweist. Die Oberflächenschichten 12 und 14 können unter elastischer Verformung der Abstandsfäden 16 gegeneinander gedrückt werden, wodurch die Wundeinlage 10 komprimiert und der Zwischenraum 18 verkleinert wird. Es ist offensichtlich, dass die Erfindung nicht auf eine Wundeinlage beschränkt ist, die nur eine erste Oberflächenschicht 12 und eine zweite Oberflächenschicht 14 aufweist, sondern das auch flächige textile Abstandsmaterialien verwendet werden können, bei welchen sich noch Zwischenschichten zwischen diesen Oberflächenschichten 12 und 14 befinden, so dass ein mehrlagiger Zwischenraum 18 gebildet wird.

Das flächige textile Abstandsmaterial der Wundeinlage 10 kann in an sich bekannter Technik hergestellt werden. Beispielsweise werden die Oberflächenschichten 12 und 14 gewirkt, wobei die Abstandsfäden 16 in die Oberflächenschichten 12 und 14 eingewirkt werden, so dass sich eine stabile Verbindung zwischen den Oberflächenschichten 12 und 14 und den Abstandsfäden 16 ergibt. Für die Oberflächenschichten 12 und 14 und die Abstandsfäden 16 werden geeignete Werkstoffe, insbesondere Kunststoffe verwendet. Das Ineinanderwirken der Fäden der Oberflächenschichten 12 und 14 und der Abstandsfäden 16 ermöglicht dabei die Verwendung unterschiedlicher Fäden für die Oberflächenschichten 12 und 14 und für die Abstandsfäden 16. Die Abstandsfäden 16 können in Bezug auf den verwendeten Werkstoff und in Bezug auf die Fadendicke entsprechend den gewünschten elastischen Eigenschaften ausgewählt werden. Für die Oberflächenschichten 12 und 14 können andere Fadenstärken, insbesondere geringere Fadenstärken verwendet werden, um eine flexible, weiche Oberflächenschicht 12 bzw. 14 zu erhalten. Auch der Werkstoff für die Oberflächeschichten 12 und 14 kann von dem Werkstoff der Abstandsfäden 16 abweichend gewählt werden.

Die Wundeinlage 10 kommt bei der später beschriebenen Anwendung zumindest mit einer der Oberflächenschichten 12 und 14 mit einer Wundoberfläche in Berührung. Zumindest eine Oberflächenschicht, im Beispiel der Figur 1 die erste Oberflächenschicht 12, die mit der Wundoberfläche in Berührung kommt, muss erfindungsgemäß folgende Kriterien erfüllen. Die Oberflächenschicht 12 muss biokompatibel sein, d. h. keine nachteiligen Reaktionen in dem Gewebe der Wunde verursachen. Weiter muss diese Oberflächenschicht 12 eine Struktur aufweisen, die einerseits Flüssigkeit durchtreten lässt und andererseits das Einwachsen von Wundgewebe verhindert. Diese Eigenschaften können in unterschiedlicher Weise erhalten werden. Es ist möglich, die Oberflächenschicht 12 aus Fäden eines biokompatiblen Werkstoffes herzustellen. Dabei kann die textile Lage der Oberflächenschicht 12 so strukturiert sein, dass die Maschenweite oder Porengröße das Durchtreten von Flüssigkeit ermöglicht, jedoch das Einwachsen von Wundgewebe weitgehend ausschließt. Es ist auch möglich, die textile Lage der Oberflächenschicht 12 mit einem biokompatiblen Werkstoff zu beschichten. Weist die textile Lage bereits eine geeignete Maschenweite bzw. Porengröße auf, so ist eine geringe Oberflächenbeschichtung ausreichend, wobei der Werkstoff der Beschichtung die Biokompatibilität bewirkt. Der Werkstoff der textilen Lage der Oberflächenschicht muss dabei diese Biokompatiblität nicht aufweisen und kann gegebenenfalls nach anderen Kriterien ausgewählt werden, wie z. B. nach den mechanischen bzw. textiltechnischen Eigenschaften. Weist die textile Lage der Oberflächenschicht 12 eine größere Maschenweite bzw. Porengröße auf, so kann die Beschichtung mit dem biokompatiblen Werkstoff zusätzlich dazu verwendet werden, die Maschenweite bzw. Porengröße auf einen solchen Wert zu reduzieren, der für das Verhindern des Einwachsens des Wundgewebes erforderlich ist.

In einer weiteren Ausführung, wie sie in Figur 1 dargestellt ist, kann die Oberflächenschicht 12 aus einer textilen Lage bestehen, die mit einer Decklage 20 kaschiert ist. Die textile Lage kann dabei eine relativ große Maschenweite aufweisen und dient im Wesentlichen als Stützlage für die Decklage 20. Bei dieser Ausführung besteht eine große Freiheit in Bezug auf die Ausbildung des Abstandsmaterials und insbesondere auch in Bezug auf die textile Lage der Oberflächenschicht 12, die als Stützlage dient. Diese Freiheit betrifft sowohl die Maschenweite, als auch die Auswahl des Werkstoffes und der Fadenstärke für die textile Lage. Die Decklage 20 weist die biokompatiblen Eigenschaften und die geforderte Struktur auf. Für die Decklage 20 kann beispielsweise eine dünne Schaumstofffolie verwendet werden, die aus einem offenporigen Kunststoffschaum besteht, z. B. aus Polyvinylalkohol. Durch die Auswahl des Kunststoffes kann optimale Biokompatiblität erreicht werden. Die Dicke der Decklage 20 und die Porengröße des Schaumstoffes können dahingehend optimiert werden, dass ein Flüssigkeitsdurchtritt möglichst wenig behindert wird, während das Einwachsen von Wundgewebe zuverlässig verhindert wird. Anstelle eines Schaumstoffes aus polymerem Kunststoff kann für die Decklage 20 auch ein textiles Material verwendet werden, welches eine geeignete Struktur aufweist.

Eine erste Anwendungsmöglichkeit ist in den Figuren 2 und 3 dargestellt. Zur Behandlung einer flächigen Wunde 22 wird eine entsprechend der Größe der Wunde 22 zugeschnittene Wundeinlage 10 auf die Wunde appliziert. Weist das Material der Wundeinlage 10 nur eine Oberflächenschicht 12 mit den erforderlichen Eigenschaften in Bezug auf die Biokompatibilität und die Struktur auf, so wird die Wundeinlage 10 mit dieser Oberflächenschicht 12 auf die Wundoberfläche aufgelegt. Anschließend werden die Wunde 22 und die Wundeinlage 10 mittels eines geeigneten Verbandsmaterials 24, z. B. einer Binde, abgedeckt. Das Verbandsmaterial 24 wird dabei so stramm angewickelt, dass die Wundeinlage 10 flächig in die Wunde 22 gepreßt wird. Die Wundeinlage 10 legt sich dadurch mit ihrer ersten Oberflächenschicht 12 dicht an die Wundoberfläche an und kommt großflächig mit der Wunde 22 in Kontakt. Der Druck des Verbandsmaterials 24 führt dabei zu einer Kompression der Wundeinlage 10, so dass die Oberflächenschichten 12 und 14 gegen die elastische Rückstellkraft der Abstandsfäden 16 zusammen gedrückt werden und der Zwischenraum 18 verkleinert wird, wie dies in Figur 2 schematisch dargestellt ist. Die elastische Rückstellkraft der Abstandsfäden 16 bewirkt einen Druck der ersten Oberflächenschicht 12 auf das Gewebe der Wunde 22, wobei das Verbandsmaterial 24 die Druckkräfte abstützt. Durch den Druck der Wundeinlage 10 auf das Gewebe der Wunde 22 wird Gewebsflüssigkeit, die sich in dem Wundgewebe befindet, aus dem Wundgewebe gedrückt. Diese Wundflüssigkeit tritt durch die erste Oberflächenschicht 12 hindurch und gelangt in den Zwischenraum 18. Durch die hierbei eintretende Kompression des Wundgewebes kann die Wundeinlage 10 unter der Rückstellkraft der Abstandsfäden 16 expandieren, wie dies in Figur 3 schematisch gezeigt ist, wodurch sich der Zwischenraum 18 vergrößert und die Gewebsflüssigkeit aufnehmen kann. Die Wundeinlage 10 kann auf diese Weise eine größere Menge Gewebsflüssigkeit aufnehmen und von dem Wundgewebe fernhalten, so dass ein Verbandswechsel weniger häufig erforderlich wird.

Die Decklage 20 kann mit Wirksubstanzen dotiert sein, z. B. mit Arzneimitteln, die die Wundheilung begünstigen, gegen Infektionskeime wirken usw. Da die Decklage 20 durch die Elastizität der Wundeinlage 10 in dichtem Kontakt mit dem Wundgewebe gebracht und gehalten wird, können die in der Decklage 20 aufgenommenen Wirksubstanzen optimal an die Wundoberfläche appliziert werden.

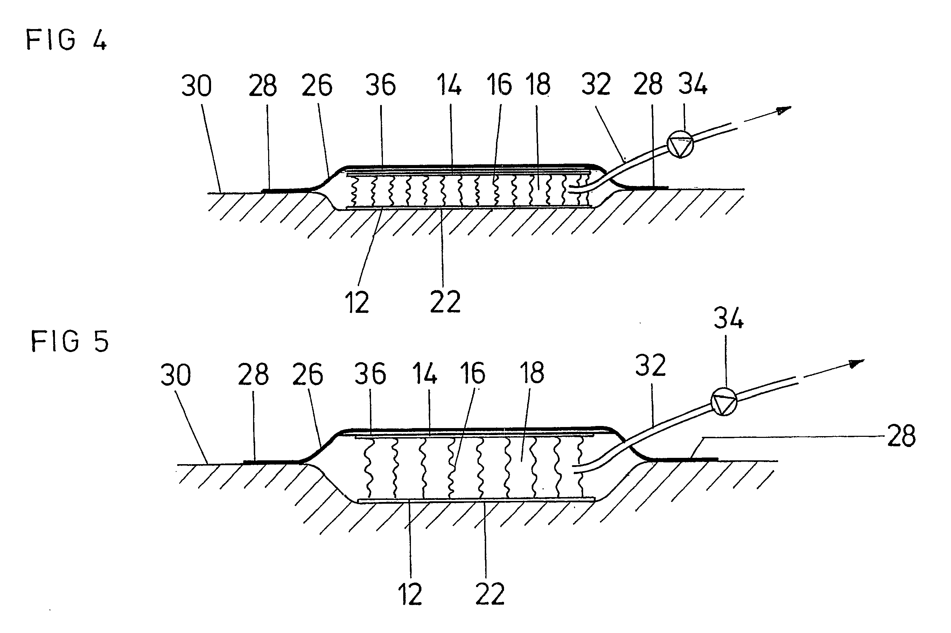

Eine zweite Anwendungsmöglichkeit wird anhand der Figuren 4 und 5 erläutert.

Auf eine Oberflächenwunde 22 wird eine entsprechend der Größe der Wunde zugeschnittene Wundeinlage 10 aufgelegt, wobei die auf der Wundoberfläche aufliegende erste Oberflächenschicht 12 in der vorstehend erläuterten Weise biokompatibel und mit einer geeigneten Struktur ausgebildet ist. Die Wunde 22 und die Wundeinlage 10 werden mit einer luftdichten Abdichtfolie 26 überdeckt. Die Abdichtfolie 26 wird außerhalb der Wunde 22 mit ihrem Randbereich 28 auf der Haut 30 dicht aufgeklebt. Die Abdichtfolie 26 kann eine verschließbare Öffnung aufweisen. Die Öffnung kann beispielsweise durch ein aufklebbares Blättchen oder ein sich nach außen öffnendes Rückschlagventil verschlossen sein. Nachdem die Wundeinlage 10 eingelegt und durch die Abdichtfolie 26 abgedichtet ist, wird die Wundeinlage 10 flächig komprimiert, in dem von außen z. B. mit der Handfläche Druck auf die Abdichtfolie 26 ausgeübt wird. Bei diesem Komprimieren kann die Luft aus der Wundeinlage 10 durch die geöffnete Öffnung der Abdichtfolie 26 bzw. durch das Rückschlagventil entweichen. Nach dem Komprimieren wird die Öffnung verschlossen, z. B. zugeklebt bzw. durch das Rückschlagventil verschlossen. Die Wundeinlage 10 liegt dann unter dem Druck der sich expandierenden Abstandsfäden 16 an der Wundoberfläche an, wie dies vorstehend beschrieben ist.

In einer Weiterbildung, wie sie in den Figuren 4 und 5 dargestellt ist, ist zusätzlich eine Schlauchleitung 32 abgedichtet unter die Abdichtfolie 26 geführt und mündet unterhalb der Abdichtfolie 26 in den Zwischenraum 18 der Wundeinlage 10. Die Schlauchleitung 32 ist mit einem Ventil 34 ausgestattet. Die Schlauchleitung 32 wird an eine Pumpe oder sonstige Unterdruckquelle angeschlossen, so dass bei geöffnetem Ventil 34 unter der Abdichtfolie 26 in der Wunde ein Unterdruck erzeugt wird. Infolge dieses Unterdrucks wird die Wundeinlage 10 gegen die Rückstellkraft der Abstandsfäden 16 komprimiert, wie dies in Figur 4 schematisch dargestellt ist. Der Unterdruck bewirkt dabei eine Zugwirkung auf die Zellen des Wundgewebes. Wird das Ventil 34 geschlossen, wie dies in Figur 5 gezeigt ist, so bewirkt die komprimierte Wundeinlage 10 einen Druck auf das Wundgewebe. Dadurch wird zum einen ein Druck auf die Zellen des Gewebes ausgeübt und zum anderen wird Wundflüssigkeit aus dem Gewebe gepresst, welches durch die Oberflächenschicht 12 in den Zwischenraum 18 der Wundeinlage 10 gelangt. Die Wundeinlage 10 expandiert dabei, wobei der sich bei der Expansion vergrößernde Zwischenraum 18 die Wundflüssigkeit aufnehmen kann. Nach einer entsprechenden Zeitspanne kann das Ventil 34 wieder geöffnet werden, um über die Schlauchleitung 32 die Wundflüssigkeit aus dem Zwischenraum 18 abzusaugen und erneut einen Unterdruck unter der Abdichtfolie 26 zu erzeugen. Dieser Vorgang kann zyklisch wiederholt werden, so dass sich die Zugwirkung auf die Zellen während der Saugperiode und die Druckwirkung auf die Zellen während der Expansion der Wundeinlage 10 abwechseln, was die Zellproliferation begünstigt.

Bei dieser Anwendung stützt sich die Wundeinlage 10 bei der Expansion an der über die Wunde gespannten Abdichtfolie 26 ab. Sofern diese Abstützwirkung der Folie unzureichend ist, kann die Abdichtfolie 26 durch eine Versteifungseinlage 36 verstärkt werden. Weiter kann in der Wundeinlage, vorzugsweise in dem Zwischenraum 18 oder an dem inneren Ende der Schlauchleitung 32 ein Drucksensor angeordnet werden, um den Druck in der Wunde unterhalb der Abdichtfolie 26 zu messen und die Absaugund Expansionsperioden entsprechend den Druckverhältnissen zu steuern. Alternativ kann in der Wundeinlage 10 auch ein Dehnungssensor angebracht sein, um den Abstand der Oberflächenschichten 12 und 14, d. h. das Maß der Kompression bzw. Expansion der Wundeinlage 10 zu messen und dem Behandlungszyklus entsprechend zu steuern.

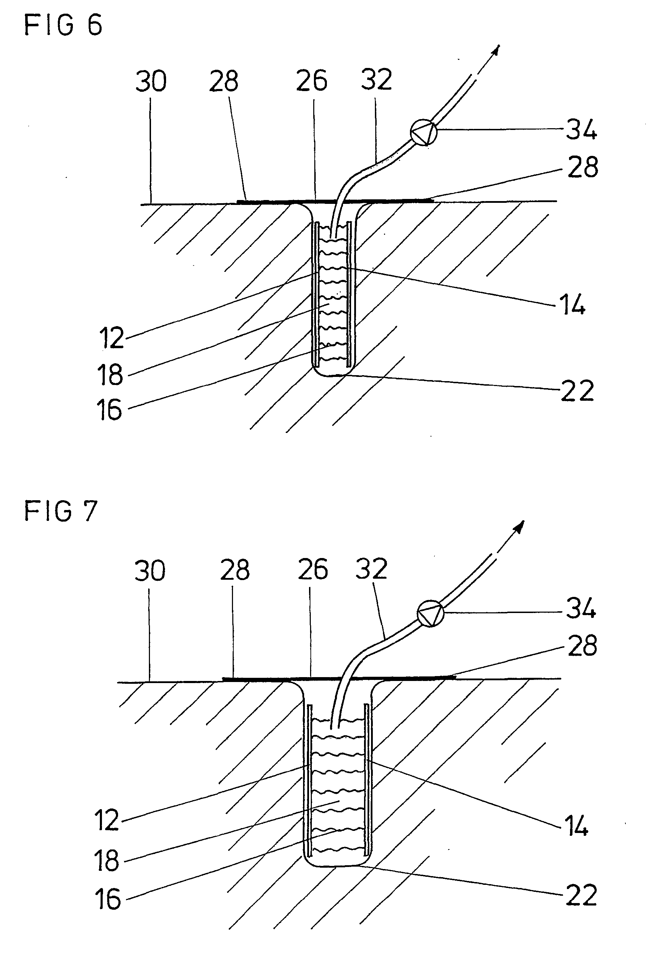

In den Figuren 6 und 7 ist eine weitere Anwendungsmöglichkeit zur Behandlung tiefer Wunden dargestellt.

Bei dieser Anwendung wird eine auf die entsprechende Größe zugeschnittene Wundeinlage 10 in eine tiefe Wundtasche 22 eingebracht. Die Wundeinlage 10 liegt dabei mit ihren beiden Oberflächenschichten 12 und 14 jeweils an den einander gegenüberliegenden Oberflächen der Wunde 22 an. Dementsprechend müssen bei dieser Verwendung beide Oberflächenschichten 12 und 14 in der vorstehend erläuterten Weise biokompatibel und mit der erforderlichen Struktur ausgebildet sein.

Nach dem Einbringen der Wundeinlage 10 in die Wunde 22 wird diese mittels einer Abdichtfolie 26 luftdicht verschlossen. Eine Schlauchleitung 32 mit Ventil 34 wird abgedichtet unter die Abdichtfolie 26 an die Wundeinlage 10 geführt. Wird bei geöffnetem Ventil 34 über die Schlauchleitung 32 abgesaugt, so entsteht in der Wunde 22 ein Unterdruck. Die einander gegenüberliegenden Wundränder werden zusammengezogen und die Wundeinlage 10 wird gegen die Kraft der Abstandsfäden 16 komprimiert, wie dies in Figur 6 dargestellt ist. Dadurch wird eine Zugwirkung auf die Zellen des Wundgewebes ausgeübt. Wird das Ventil 34 geschlossen, so übt die komprimierte Wundeinlage 10 einen Druck auf die einander gegenüberliegenden Wundoberflächen aus. Dadurch wird ein Druck auf die Zellen des Wundgewebes ausgeübt und Wundflüssigkeit wird ausgequetscht und in dem sich erweiternden Zwischenraum 18 aufgenommen. Die Wundeinlage 10 stützt sich dabei an den einander gegenüberliegenden Wundflächen ab.

In den Ausführungen der Figuren 4 bis 7 kann in vorteilhafter Weise auch eine Instillationsbehandlung der Wunde 22 durchgeführt werden. Hierzu kann eine Behandlungsflüssigkeit entweder über die Schlauchleitung 32 oder über eine weitere unter die Abdichtfolie 26 geführte Zuführleitung eingeleitet werden. Damit ergeben sich zahlreiche weitere Behandlungsmöglichkeiten. Es kann Behandlungsflüssigkeit in den Zwischenraum 18 eingeleitet werden, die durch die poröse Struktur der Oberflächenschicht 12 bzw. 12 und 14 an die Wundoberfläche gebracht wird. Diese Behandlungsflüssigkeit und die abgesonderte Wundflüssigkeit können wiederum über die Schlauchleitung 32 abgesaugt werden. Bei dieser Behandlung muss die Wundeinlage 10 nicht komprimiert werden oder nur soweit komprimiert werden, dass sie mit der Oberflächenschicht 12 bzw. 14 in innigen Kontakt mit der Wundoberfläche kommt. Der großvolumige freie Zwischenraum 18 gewährleistet dabei, dass die Behandlungsflüssigkeit unbehindert über die gesamte Wundoberfläche appliziert wird und ein ungehindertes Absaugen möglich ist, ohne dass die Gefahr eines Zusetzens oder Verklebens durch Gerinselbildungen oder dergleichen besteht. Weiter kann diese Instillation auch mit einer Unterdruckerzeugung in der vorstehend beschriebenen Weise kombiniert werden. Hierbei kann in gesteuerten Zeitintervallen Behandlungsflüssigkeit an die Wundoberfläche appliziert werden, ein Unterdruck in der Wunde erzeugt werden und ein Druck durch die expandierende Wundeinlage 10 auf das Wundgewebe ausgeübt werden.

### Bezugszeichenliste

- 10: Wundeinlage
- 12: erste Oberflächenschicht
- 14: zweite Oberflächenschicht
- 16: Abstandsfäden
- 18: Zwischenraum
- 20: Decklage
- 22: Wunde
- 24: Verbandsmaterial
- 26: Abdichtfolie
- 28: Randbereich
- 30: Haut
- 32: Schlauchleitung
- 34: Ventil
- 36: Versteifungseinlage

## Patentansprüche

1. Vorrichtung zur Behandlung von Wunden, mit einer mit der Wundoberfläche in Berührung kommenden Wundeinlage und einer Abdeckung zur Überdeckung der Wundfläche und der Wundeinlage, wobei die Abdeckung eine Abdichtfolie (26) ist, die an der die Wunde (22) umgebenden Haut (30) abdichtend befestigbar ist und wobei wenigstens eine Schlauchleitung (32) abgedichtet unter der Abdichtfolie (26) herausführbar ist,
**dadurch gekennzeichnet, dass** die Wundeinlage (10) aus einem flächigen textilen Abstandsmaterial besteht, welches wenigstens eine erste Oberflächenschicht (12), eine zweite Oberflächenschicht (14) und einen Zwischenraum (18) zwischen diesen Oberflächenschichten (12, 14) aufweist, wobei wenigstens die erste Oberflächenschicht (12) eine biokompatible Oberfläche und eine Struktur aufweist, die das Durchtreten von Flüssigkeit ermöglicht und das Einwachsen des Wundgewebes verhindert, und wobei in dem Zwischenraum (18) Abstandsfäden (16) angeordnet sind, die die erste Oberflächenschicht (12) und die zweite Oberflächenschicht (14) elastisch federnd beabstandet halten, und dass die wenigstens eine Schlauchleitung (32) mit dem Zwischenraum (18) kommuniziert.

2. Vorrichtung nach Anspruch 1,
**dadurch gekennzeichnet, dass** die Abstandsfäden (16) aus einem entsprechend den mechanischen Eigenschaften gewählten Werkstoff bestehen und dass wenigstens die erste Oberflächenschicht (12) aus einem biokompatiblen Werkstoff besteht.

3. Vorrichtung nach Anspruch 2,
**dadurch gekennzeichnet, dass** wenigstens die erste Oberflächenschicht (12) aus einer textilen Lage geringer Dicke und kleiner Maschenweite aus Fäden des biokoznpatiblen Werkstoffs besteht, in welche die Abstandsfäden (16) eingewirkt sind.

4. Vorrichtung nach Anspruch 1,
**dadurch gekennzeichnet, dass** wenigstens die erste Oberflächenschicht (12) mit einem biokompatiblen Werkstoff beschichtet ist.

5. Vorrichtung nach Anspruch 1,
**dadurch gekennzeichnet, dass** wenigstens die erste Oberflächenschicht (12) mit einer Decklage (20) aus einem biokompatiblen Werkstoff kaschiert ist.

6. Vorrichtung nach Anspruch 5,
**dadurch gekennzeichnet, dass** wenigstens die erste Oberflächenschicht (12) mit den Abstandsfäden (16) gewirkt ist und als Stützlage für die Decklage (20) dient.

7. Vorrichtung nach Anspruch 5 oder 6,
**dadurch gekennzeichnet, dass** die Decklage (20) aus einem Schaumstoff oder einem Textilmaterial kleiner Porengröße besteht.

8. Vorrichtung nach einem der Ansprüche 1 bis 7,
**dadurch gekennzeichnet , dass** der biokompatible Werkstoff ein polymerer Kunststoff ist, insbesondere ein Polyvinylalkohol.

9. Vorrichtung nach Anspruch 1,
**dadurch gekennzeichnet, dass** die wenigstens eine Schlauchleitung (32) als Absaugleitung an eine Unterdruckquelle anschließbar ist.

10. Vorrichtung nach Anspruch 9,
**dadurch gekennzeichnet, dass** wenigstens eine weitere Schlauchleitung als Zuführleitung für vorzugsweise flüssige Behandlungsmittel ausgebildet ist.

11. Vorrichtung nach Anspruch 9 oder 10,
**dadurch gekennzeichnet, dass** die wenigstens eine Schlauchleitung (32) zum Absaugen und/oder gegebenenfalls Zuführen gesteuert geöffnet und geschlossen werden kann.

12. Vorrichtung nach Anspruch 11,
**dadurch gekennzeichnet, dass** die Steuerung in vorgebbaren Zeitintervalle erfolgt.

13. Vorrichtung nach Anspruch 11,
**dadurch gekennzeichnet, dass** die Steuerung in Abhängigkeit von dem Druck in der Wunde (22) erfolgt.

14. Vorrichtung nach Anspruch 13,
**dadurch gekennzeichnet, dass** in der Wundeinlage (10) ein Drucksensor oder ein Dehnungssensor angeordnet ist.

15. Vorrichtung nach einem der Ansprüche 5 bis 7,
**dadurch gekennzeichnet, dass** die Decklage (20) einen an die Wunde zu applizierenden Wirkstoff enthalt.

## Claims

1. An apparatus for the treatment of wounds, with a wound dressing coming into contact with the wound surface and a cover for covering the wound area and the wound dressing, wherein the cover has a sealing film (26) which is capable of being fixed in a sealing manner to the skin (30) surrounding the wound (22), and wherein at least one hose line (32) is capable of being brought out in a sealed manner under the sealing film (26), **characterized in that** the wound dressing (10) consists of a flat textile spacer material which has at least one first surface layer (12), one second surface layer (14) and one intermediate space (18) between these surface layers (12, 14), wherein at least the first surface layer (12) has a biocompatible surface and a structure which allows the passage of liquid and prevents the growth of wound tissue, and wherein spacer threads (16), which keep the first surface layer (12) and the second surface layer (14) at a distance in a resilient manner, are arranged in the intermediate space (18), and the at least one hose line (32) communicates with the intermediate space (18).

2. An apparatus according to claim 1, **characterized in that** the spacer threads (16) consist of a material selected according to the mechanical properties, and at least the first surface layer (12) consists of a biocompatible material.

3. An apparatus according to claim 2, **characterized in that** at least the first surface layer (12) of a textile layer of slight thickness and small mesh width consists of threads of the biocompatible material into which the spacer threads (16) are knitted.

4. An apparatus according to claim 1, **characterized in that** at least the first surface layer (12) is coated with a biocompatible material.

5. An apparatus according to claim 1, **characterized in that** at least the first surface layer (12) is laminated with a cover layer (20) of a biocompatible material.

6. An apparatus according to claim 5, **characterized in that** at least the first surface layer (12) is knitted with the spacer threads (16) and acts as a support layer for the cover layer (20).

7. An apparatus according to claim 5 or 6, **characterized in that** the cover layer (20) consists of a foam material or a textile material of small pore size.

8. An apparatus according to any one of claims 1 to 7, **characterized in that** the biocompatible material is a polymer plastic, in particular a polyvinyl alcohol.

9. An apparatus according to claim 1, **characterized in that** the at least one hose line (32) is capable of being attached as a suction line to an under-pressure source.

10. An apparatus according to claim 9, **characterized in that** at least one further hose line is designed in the form of a supply line for preferably liquid treatment agents.

11. An apparatus according to claim 9 or 10, **characterized in that** the at least one hose line (32) can be opened and closed in a controlled manner for suction and/or optionally supply.

12. An apparatus according to claim 11, **characterized in that** the control is carried out at pre-set time intervals.

13. An apparatus according to claim 11, **characterized in that** the control is carried out in a manner dependent upon the pressure in the wound (22).

14. An apparatus according to claim 13, **characterized in that** a pressure sensor or a strain sensor is arranged in the wound dressing (10).

15. An apparatus according to any one of claims 5 to 7, **characterized in that** the cover layer (20) contains an active substance to be applied to the wound.

## Revendications

1. Dispositif de traitement de plaies comprenant une garniture de plaie venant en contact avec la surface de la plaie et un revêtement pour recouvrir la surface de la plaie et la garniture de plaie, le revêtement étant de une feuille d'étanchéité (26) pouvant être fixée de manière étanche à la peau (30) entourant la plaie (22), et, au moins une conduite tubulaire (32) pouvant sortir de façon étanche au-dessous de la feuille d'étanchéité (26),
**caractérisé en ce que**
la garniture de plaies (10) est réalisée en un matériau d'écartement textile plan qui comporte au moins une première couche surfacique (12), une seconde couche surfacique (14) et un volume intermédiaire (18) situé entre ces couches surfaciques (12, 14), au moins la première couche surfacique (12) comprenant une surface biocompatible et une structure permettant le passage de liquides et empêchant la croissance du tissu de la plaie, et, le volume intermédiaire (18) renfermant des fibres d'écartement (16) qui maintiennent élastiquement à distance la première couche surfacique (12) et la seconde couche surfacique (14), et, la conduite tubulaire (32) communiquant avec le volume intermédiaire (18).

2. Dispositif conforme à la revendication 1,
**caractérisé en ce que**
les fibres d'écartement (16) sont réalisées en un matériau choisi en fonction de ses propriétés mécaniques, et au moins la première couche surfacique (12) est réalisée en un matériau biocompatible.

3. Dispositif conforme à la revendication 2,
**caractérisé en ce qu'**
au moins la première couche surfacique (12) est constituée d'une couche textile de faible épaisseur et ayant une faible ouverture de mailles réalisée en fibres du matériau biocompatible dans laquelle sont tricotées les fibres d'écartement (16).

4. Dispositif conforme à la revendication 1,
**caractérisé en ce qu'**
au moins la première couche surfacique (12) est revêtue d'un matériau biocompatible.

5. Dispositif conforme à la revendication 1,
**caractérisé en ce qu'**
au moins la première couche surfacique (12) est contrecollée avec une couche de couverture (20) en un matériau biocompatible.

6. Dispositif conforme à la revendication 5,
**caractérisé en ce qu'**
au moins la première couche surfacique (12) est tricotée avec les fibres d'écartement (16) et sert de couche d'appui pour la couche de couverture (20).

7. Dispositif conforme à la revendication 5 ou 6,
**caractérisé en ce que**
la couche de couverture (20) est constituée d'un matériau cellulaire ou d'un matériau textile ayant une faible dimension de pores.

8. Dispositif conforme à l'une des revendications 1 à 7,
**caractérisé en ce que**
le matériau biocompatible est un matériau synthétique polymère, en particulier un alcool polyvinylique.

9. Dispositif conforme à la revendication 1,
**caractérisé en ce que**
la conduite tubulaire (32) est constituée d'une conduite d'aspiration reliée à une source de dépression.

10. Dispositif conforme à la revendication 9,
**caractérisé en ce qu'**
il est prévu au moins une autre conduite tubulaire constituant une conduite d'introduction d'agents de traitement, de préférence liquide.

11. Dispositif conforme à la revendication 9 ou 10,
**caractérisé en ce que**
la conduite tubulaire (32) peut être ouverte ou fermée de manière commandée pour permettre d'aspirer et/ou le cas échéant d'introduire des produits.

12. Dispositif conforme à la revendication 11,
**caractérisé en ce que**
la commande s'effectue à des intervalles de temps pouvant être prédéfinis.

13. Dispositif conforme à la revendication 11,
**caractérisé en ce que**
la commande s'effectue en fonction de la pression dans la plaie (22).

14. Dispositif conforme à la revendication 13,
**caractérisé en ce que**
dans la garniture de plaie (10) est monté un capteur de pression ou un capteur de dilatation.

15. Dispositif conforme à l'une des revendications 5 à 7,
**caractérisé en ce que**
la couche de couverture (20) renferme une matière active à appliquer sur la plaie.
